# EUROPEAN PATENT SPECIFICATION

(11) **EP 2 064 337 B1**
(45) Date of publication and mention of the grant of the patent: **18.07.2012**
(21) Application number: 07838397.3
(22) Date of filing: 18.09.2007
(51) Int. Cl.: C12N 15/10

(54) **A FACS-AND REPORTER PROTEIN-BASED SYSTEM FOR HIGH THROUGHPUT DEVELOPMENT OF THERAPEUTIC PROTEINS**
FACS- UND REPORTERPROTEIN-BASIERTES SYSTEM ZUR ENTWICKLUNG THERAPEUTISCHER PROTEINE MIT HOHEM DURCHSATZ
SYSTÈME À BASE DE FACS ET DE PROTÉINE REPORTEUR POUR UN DÉVELOPPEMENT HAUT DÉBIT DE PROTÉINES THÉRAPEUTIQUES

(30) Priority: 20.09.2006 US 846223 P; 27.09.2006 US 847705 P
(43) Date of publication of application: 03.06.2009
(73) Proprietor: Genzyme Corporation, Cambridge, MA 02142 (US)
(72) Inventor: DEMARIA, Christine, Franklin, MA 02038 (US); ESTES, Scott, Framingham, MA 01701 (US); KAREY, Kenneth, P., Bolton, MA 01740 (US); CAIRNS, Victor, Worcester, MA 01606 (US)
(74) Representative: Adams, Harvey Vaughan John
(86) International application number: PCT/US2007/020180
(87) International publication number: WO 2008/036255

(56) References cited:
- WO-A-01/57212
- GAINS P ET AL: "PIRES-CD4T, A DICISTRONIC EXPRESSION VECTOR FOR MACS- OR FACS-BASED SELECTION OF TRANSFECTED CELLS" BIOTECHNIQUES, INFORMA LIFE SCIENCES PUBLISHING, WESTBOROUGH, MA, US, vol. 26, no. 4, April 1999 (1999-04), pages 683-688, XP001010486 ISSN: 0736-6205
- MEDIN J A ET AL: "A BICISTRONIC THERAPEUTIC RETROVIRAL VECTOR ENABLES SORTING OF TRANSDUCED CD34+ CELLS AND CORRECTS THE ENZYME DEFICIENCY IN CELLS FROM GAUCHER PATIENTS" BLOOD, W.B.SAUNDERS COMPANY, ORLANDO, FL, US, vol. 87, no. 5, 1 March 1996 (1996-03-01), pages 1754-1762, XP001030719 ISSN: 0006-4971
- DEMARIA CHRISTINE T ET AL: "Accelerated clone selection for recombinant CHO cells using a FACS-based high-throughput screen" BIOTECHNOLOGY PROGRESS, vol. 23, no. 2, March 2007 (2007-03), pages 465-472, XP002477648 ISSN: 8756-7938
- YOSHIKAWA T ET AL: "Flow cytometry: An improved method for the selection of highly productive gene-amplified CHO cells using flow cytometry" BIOTECHNOLOGY AND BIOENGINEERING, WILEY & SONS, HOBOKEN, NJ, US, vol. 74, no. 5, 5 September 2001 (2001-09-05), pages 435-442, XP002350920 ISSN: 0006-3592

## Description

### RELATED APPLICATIONS

This application claims the benefit of U.S. Provisional Application No. 60/847,705, filed on September 27, 2006, and U.S. Provisional Application No. 60/846,223, filed on September 20, 2006. The entire teachings of the above applications are incorporated herein by reference.

### FIELD OF THE INVENTION

This disclosure generally relates to high throughput systems for identifying high producing clones useful for the production of therapeutic and diagnostic proteins.

### BACKGROUND OF THE INVENTION

Commercial production of a therapeutic protein requires a stable, high expressing recombinant cell line. Prior art methods have relied on dyhydrofolate reductase deficient (DHFR) Chinese Hamster Ovary (CHO) cell lines to manufacture clonal cell lines that produce the protein(s). High producing clones are obtained after repeated selection based amplification of transfected pools of cells using agents such as methotrexate (MTX). This process is time-consuming and labor intensive and does not specifically identify clones that will produce proteins at high levels.

Flow cytometry or FACS has been used to improve the development and selection of high production CHO cell lines. This technology has enabled rapid identification and isolation of high production clones from a heterogeneous population of transfected cells thereby decreasing the labor and time associated with random cloning methods. It can identify desired cells without the need for repeated MTX amplification of pools.

Once single cell clones have been isolated via a FACS-based sorting method, however, screening an adequate number of clones to isolate a stable, high producing cell line still remains a time-consuming process. Therefore, a highly efficient and accurate screening method is extremely advantageous at this stage of cell line development. In addition, implementing a screen at the 96-well plate stage of clone development, for example, is desirable as it focuses further expansion only on the clones with high productivity. Analysis of cell culture media harvests is commonly used to identify clones secreting high levels of a therapeutic protein. However, this method is not optimal as it does not account for differences in either cell density or media volume between wells. As such, it may not accurately predict the clones with high specific productivity and high titer. Also, effort must often be spent to develop and optimize a new assay for each new therapeutic protein of interest.

Thus, a need exists in the art for compositions and methods to screen clonal populations to select recombinant cell lines that stably produce high levels of the protein of interest. This invention satisfies this need and provides related advantages as well.

WO 01/57212 discloses a method of identifying a host cell that exhibits regulated expression of a test gene by providing a plurality of host cells, each host cell comprising a polynucleotide, the polynucleotide comprising in order a regulatable promoter; a test gene; an IRES sequence; and a surface marker coding sequence, where the surface marker comprises a secretion signal sequence, a detectable label protein, and a membrane anchor; wherein expression of said test gene also results in expression of said surface marker; inducing said promoter; and selecting a host cell that displays said surface marker on its surface.

GAINS P et al: "pIRES-CD4t, a dicistronic expression vector for MACS- or FACS-based selection of transfected cells" BIOTECHNIQUES, Vol. 26, no.4, April 1999, pages 683-688, discloses a plasmid vector that directs the co-expression of heterologous cDNAs and a 3'-positioned cassette encoding a truncated CD4 marker. An encephalomyocarditis virus internal ribosomal entry site (IRES) mediates translational initiation from this 3' cassette, and a cytomegalovirus promoter drives dicistronic transcript expression. MEDIN J A et al: "A bicistronic therapeutic retroviral vector enables sorting of transduced CD34+ cells and corrects the enzyme deficiency in cells from Gaucher patients" BLOOD, vol.87, no. 5, 1 March 1996, pages 1754-1762, discloses a bicistronic recombinant retrovirus that delivers both a therapeutic glucocerebrosidase (GC) cDNA for the treatmet of Gaucher disease, and a small murine cell surface antigen (heat-stable antigen [HSA]) as a selectable marker.

### SUMMARY OF THE INVENTION

The present invention is as defined in the appended claims.

Methods are disclosed to identify, select and produce a population of recombinant eukaryotic host cells that stably express a polypeptide of interest ("target polypeptide") at high levels suitable for large scale production of the target polypeptide. The eukaryotic host cell used in these methods contains or comprises a recombinant polynucleotide, wherein the recombinant polynucleotide comprises a promoter element; a polynucleotide encoding a cell surface marker polypeptide; a polynucleotide encoding a target polypeptide; and a polynucleotide having the biological activity of an internal ribosome entry site (IRES) polynucleotide, and wherein the IRES polynucleotide is located within the recombinant polynucleotide such that the cell surface marker polynucleotide and the target polynucleotide are transcribed on the same mRNA. In an alternate embodiment, the recombinant polynucleotide includes an alternate start codon for translation initiation. The alternate start codon can be in addition to the IRES element or alternatively, be substituted for the IRES element. Recombinant host cells are bound by an agent which binds, directly or indirectly the cell surface marker, and such cells are then selected and isolated. The selected and isolated cells are then prepared into one or more clonal populations and cultured for a period of time. The clonal populations are then analyzed by detecting the level of the cell surface marker expression on said clonal population and one or more clonal populations with high expression levels of the cell surface marker are selected. The selected clonal populations with high expression levels of the cell surface marker indicate clonal populations with stable and high expression levels of the target polypeptide. The cells selected by these methods can be further cultured under conditions that enable the production of the target polypeptide that also is present in the host cell. The target polypeptide can be further isolated from the cells or cell culture media as appropriate.

Also disclosed herein are products produced by the above-noted methods and assemblies of components necessary to conduct the methods

### BRIEF DESCRIPTION OF THE DRAWINGS

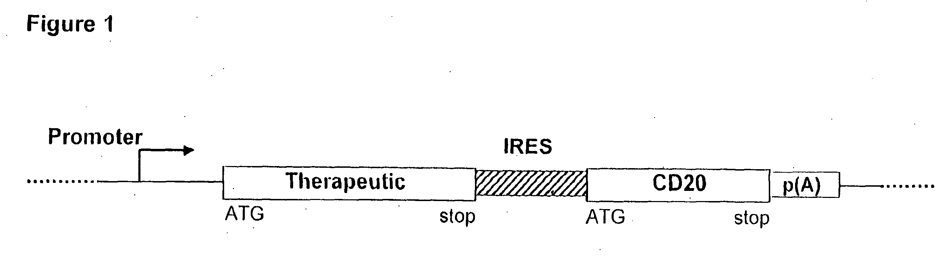

**Figure 1** is a diagram of a gene expression cassette. In an embodiment of this invention, the DNAs encoding the therapeutic polypeptide and the CD20 polypeptide were linked by an IRES, so that they were transcribed in the same mRNA but were translated independently.

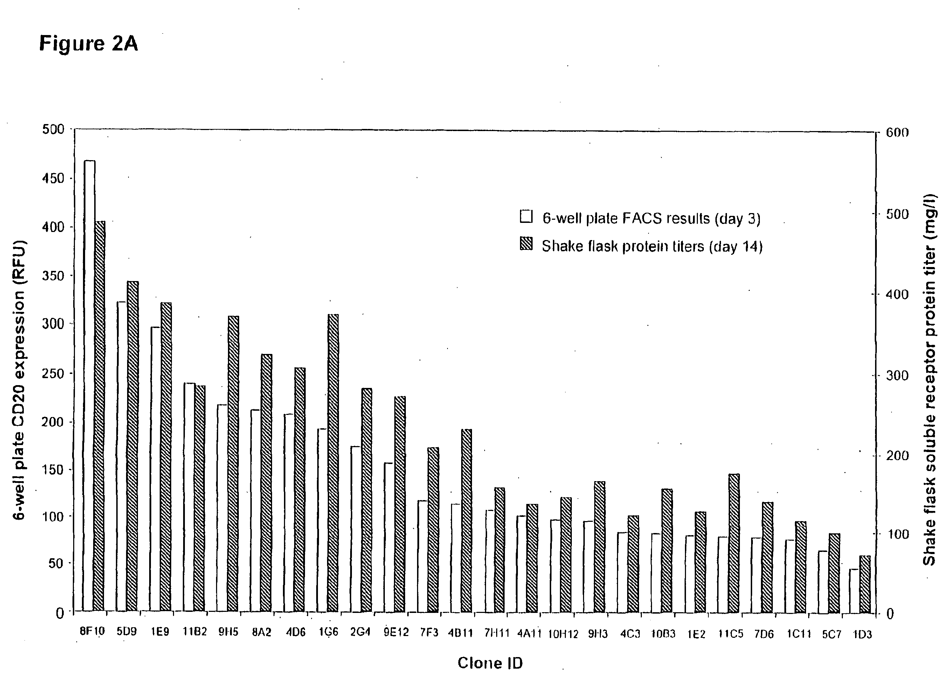

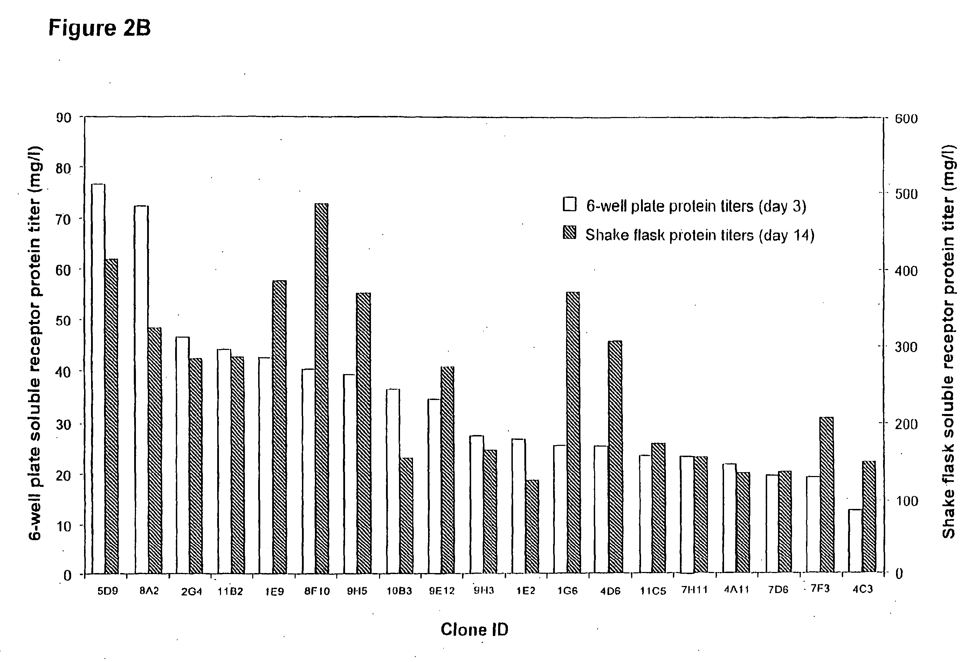

**Figures 2A** **and** **2B** show that the CD20-based FACS screen accurately identified clones with high soluble receptor titers. Cells were seeded in 6-well plates at 5 x 10⁵ cells/ml, and were assayed at day 3 by both FACS analysis (Figure 2A) of cell surface CD20 expression and protein A HPLC analysis (Figure 2B) of conditioned media (open bars in each graph). The clones were scaled up to 125-ml shake flasks and assayed for protein titer (hatched bars in each graph). For FACS results, each bar represented a measure of the geometric mean fluorescence intensity, quantified in relative fluorescence units (RFU).

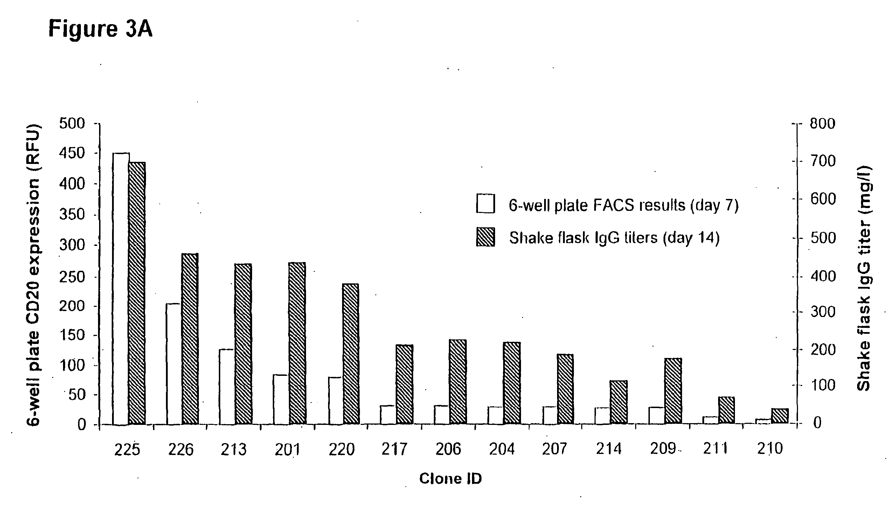

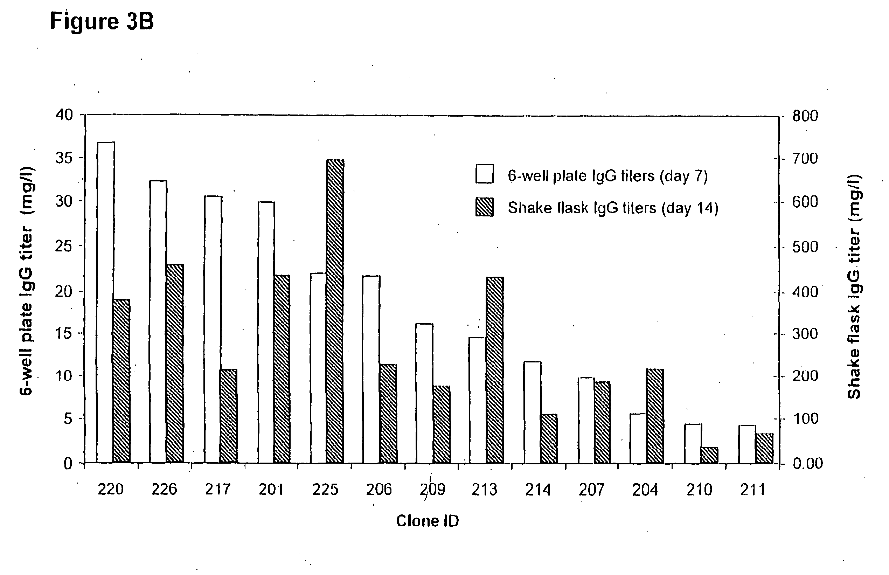

**Figures 3A** **and** **3B** show that the CD20-based FACS screen accurately identified clones with high antibody titers. Upon reaching 80-90% confluence in a 96-well plate, the total cells from each well were seeded into one well of a 6-well plate. After 7 days, the cells were assayed by both FACS analysis (Figure 3A) of cell surface CD20 expression and protein A HPLC analysis (Figure 3B) of conditioned media (open bars in each graph). The clones were scaled up to 125-ml shake flasks and assayed for antibody titer (hatched bars in each graph). In these clones, CD20 was co-expressed with the antibody heavy chain, while the light chain was expressed separately. For FACS results, each bar represented a measure of the geometric mean fluorescence intensity, quantified in relative fluorescence units (RFU).

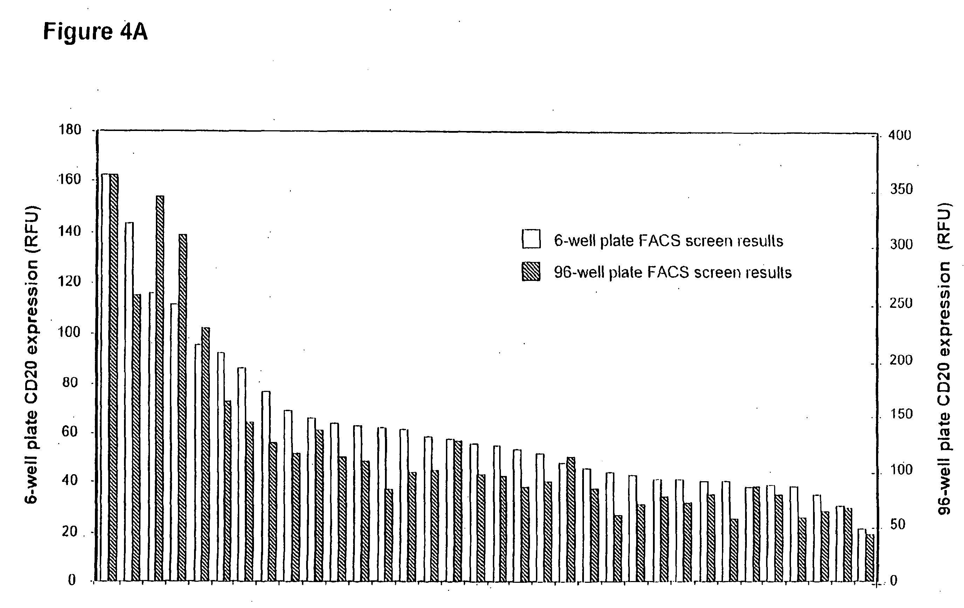

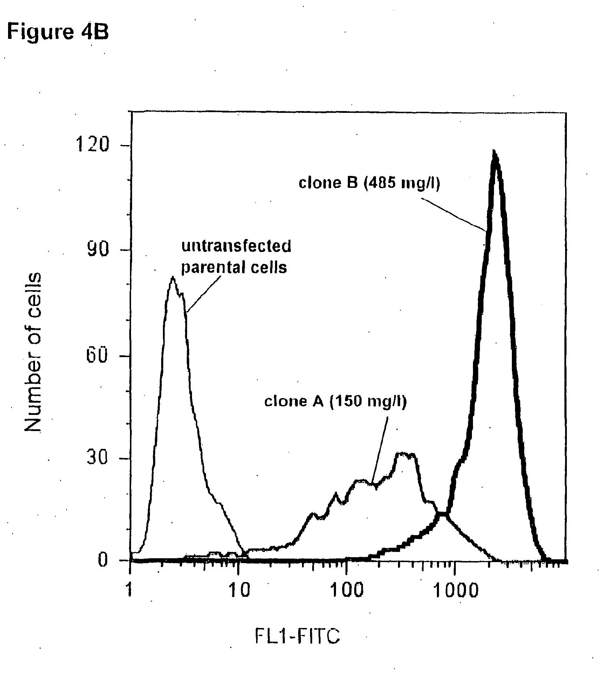

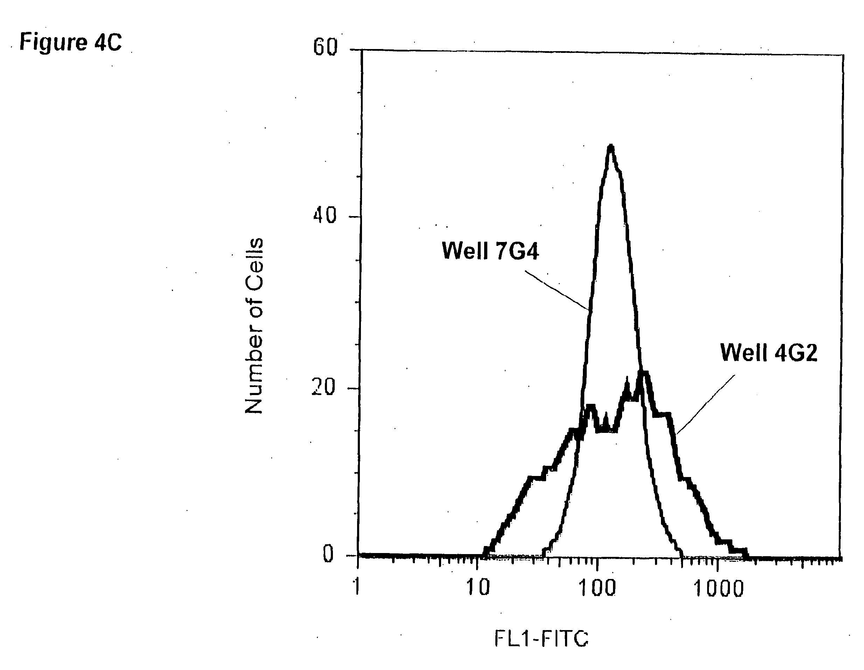

**Figures 4A to 4C** show quantitative and qualitative FACS screening of clones at the 96-well plate stage. In Figure 4A, CHO clones expressing a soluble receptor were screened by FACS at both the 96-well plate and 6-well plate stages. Clone ranking by the 96-well FACS screen (hatched bars) was very similar to that of the 6-well plate FACS screen (open bars). Each bar represented a measure of the geometric mean fluorescence intensity, quantified in relative fluorescence units (RFU). In Figure 4B, the 96-well plate FACS profiles of two representative clones expressing a soluble receptor showed the correlation between FACS data and the final protein titers in unfed batch shake flask cultures. In Figure 4C, for cells expressing an IgG, the FACS profiles for two wells of a 96-well plate were overlaid to illustrate the qualitative difference between cell populations with nearly identical fluorescence intensities (127.5 and 127.1 RFU for wells 4G2 and 7G4, respectively).

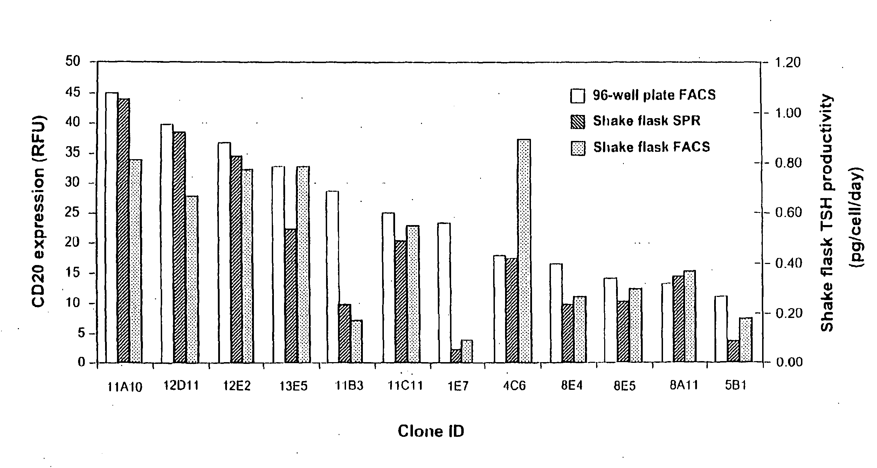

**Figure 5** shows that clonal declines in therapeutic protein productivity paralleled declines in CD20 expression. Clones were stained with PE-conjugated anti-CD20 antibody at the 96-well plate stage; these results were ranked in descending order (open bars). Clones were then scaled up to 125-ml shake flasks and assayed for specific productivity (SPR) at 24 hours (hatched bars). At 24 hours of shake flask culture, cells were also analyzed by FACS after staining with FITC-conjugated anti-CD20 antibody (dotted bars). For FACS results, each bar represented a measure of the geometric mean fluorescence intensity, quantified in relative fluorescence units (RFU)

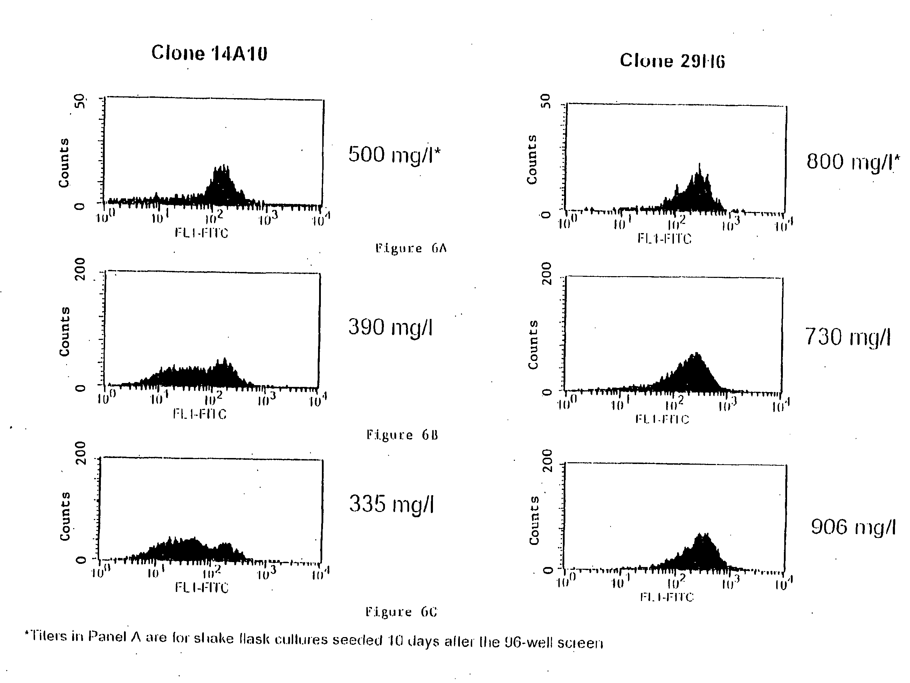

**Figures 6A to 6C** show that the CD20-based FACS clone screen identified unstable clones during scale up. Representative FACS screen and IgG titer results are shown for two clones that were selected for further development by the 96-well plate FACS screen. Clones were scaled up to shake flasks and seeded into unfed batch cultures 10 days after the 96-well screen. Figure 6A shows 96-well plate FACS screen results and initial unfed shake flask titers. Figure 6B shows shake flask FACS results (day 3) and IgG titers (day 14) after 3 additional weeks in culture. Figure 6C shows shake flask FACS results (day 3) and IgG titers (day 14) after another 3 weeks in culture.

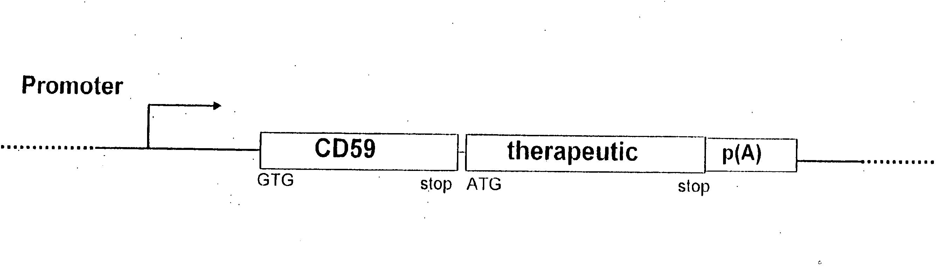

**Figure 7** is a diagram of a gene expression cassette using an alternate start codon. In one example of the mammalian expression vector used, the DNA encoding the CD59 polypeptide contained the alternate start codon GTG for translation initiation. The DNA encoding the therapeutic polypeptide contained an ATG start codon for translation initiation and was located downstream of the DNA encoding the CD59 protein. The DNA sequences encoding each polypeptide were transcribed in the same mRNA, and 5'-cap mediated translation initiated at either the GTG or the ATG start codon, with the former being less efficient and therefore less likely to have occurred.

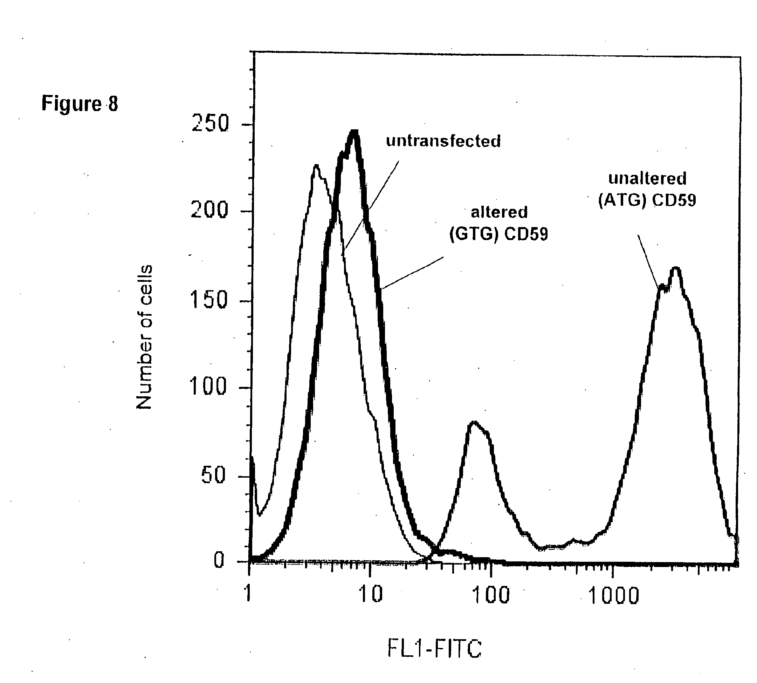

**Figure 8** illustrates that altering the ATG start codon resulted in decreased CD59 polypeptide expression. CHO cells were transfected with expression vectors encoding CD59 containing either an ATG start codon or a GTG start codon. For both DNA sequences, the internal ATG triplets were changed to prevent internal translation initiation. Stably transfected cells were grown as pools and analyzed for CD59 expression by incubation with a FITC-conjugated anti-CD59 antibody. FACS analysis of two representative pools expressing CD59 was performed. For a pool expressing CD59 with an unaltered start codon (ATG), 99% of the population was positive for CD59 expression. In contrast, for a pool expressing CD59 with an altered start codon (GTG), only 4% of the population was positive for CD59 expression. The percent of CD59 positive cells was quantified as that above background (untransfected cells).

### DETAILED DESCRIPTION

As used herein, certain terms have the following defined meanings.

### Definitions

The practice of the present disclosure will employ, unless otherwise indicated, conventional techniques of immunology, molecular biology, microbiology, cell biology and recombinant DNA, which are within the skill of the art. See, e.g., Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I: Freshney, ed. (1987)).

As used in the specification and claims, the singular form "a", "an" and "the" include plural references unless the context clearly dictates otherwise. For example, the term "a cell" includes a plurality of cells, including mixtures thereof.

As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention. Embodiments defined by each of these transition terms are within the scope of this disclosure.

As used herein, the term "polynucleotide" intends a polymeric form of nucleotides of any length, examples of which include, but are not limited to a gene or gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs.

As used herein, the term "IRES" or "a polynucleotide having the biological activity of IRES" is intended to include any molecule such as a polynucleotide or its reverse transcript which is able to initiate translation of the polynucleotide operatively linked to the IRES without the benefit of a cap site in a eukaryotic cell. An IRES or a polynucleotide having the biological activity of IRES can be identical to sequences found in nature, such as the picornavirus IRES, or they can be non-naturally or non-native sequences that perform the same function when introduced into a suitable host cell.

As used herein, the term "alternate start codon" is intended to include any non-ATG polynucleotide (typically a triplet) that functions as a start site for translation initiation with reduced efficiency relative to that of an ATG start codon. See, for example, Figure 8. Naturally occurring alternate start codon usage is known in the art and described for example in Kozak (1991) J. Cell Biol. 115(4): 887-903; Mehdi et al. (1990) Gene 91:173-178; Kozak (1989) Mol. Cell. Biol. 9(11): 5073-5080. In general, alternate start codons have decreased translation efficiencies compared to that of an ATG; for example, the alternate start codon GTG may have 3-5% translation efficiency compared to that of an ATG (100%). The translation efficiency of an alternate start codon can also be affected by its sequence context; for example, an optimal Kozak consensus sequence is reported to have a positive effect on translation initiation at alternate start codons (Mehdi et al. (1990) Gene 91:173-178; Kozak (1989) Mol. Cell. Biol. 9(11): 5073-5080). The complete Kozak consensus sequence is GCCRCCATGG, where the start codon ATG is underlined, the A of the ATG start codon is designated as the +1 position, and "R" at position -3 is a purine (A or G). The two most highly conserved positions are a purine, preferably an A, at -3 and a G at +4 (Kozak (1991) J Cell Biol 115(4): 887-903). Alternate start codon usage is described for attenuated expression of a selectable marker in U.S. Patent Publication 2006/0172382 and U.S. Patent Publication 2006/0141577. One of skill in the art will recognize that the sequences described herein as DNA will have correlative sequences as RNA molecules e.g., DNA sequence ATG, for example, would correspond to RNA sequence AUG.

As used herein, a "high expression level" refers to a level of expression that is higher than the expression level of at least 50, 70, 80, 90, 95 or 99% of the total cells analyzed.

### Assemblies, Cells & Methods

To improve the accuracy and throughput of clone screening and to identify cells that stably express a target polypeptide at high levels, the disclosure provides methods comprising:
(a) contacting at least one recombinant eukaryotic host cell containing a recombinant polynucleotide, wherein the recombinant polynucleotide comprises a promoter element; a polynucleotide encoding a cell surface marker polypeptide; a polynucleotide encoding a target polypeptide; and a polynucleotide having the biological activity of an internal ribosome entry site (IRES) polynucleotide or an alternate start codon, and wherein the IRES polynucleotide or the alternate start codon is located within the recombinant polynucleotide such that the cell surface marker polynucleotide and the target polynucleotide are transcribed on the same mRNA, and wherein the culturing is under conditions such that the cell surface marker polypeptide is expressed on the surface of the host cell, with a detectable agent that recognizes and directly or indirectly binds the cell surface marker, if present, on the surface of the host cell, said contacting under conditions that favor binding of the agent with the cell surface marker;
(b) selecting any host cell that is directly or indirectly bound to the agent
(c) preparing one or more clonal populations of the host cells from step (b); and
(d) analyzing one or more clonal populations from step (c) by detecting the level of the cell surface marker expression on said clonal population and selecting one or more clonal populations with a high expression level of the cell surface marker, thereby selecting one or more clonal populations stably expressing the target polypeptide.

In one embodiment, step (b) and/or (d) is by performed fluorescence activated cell sorting.

In one embodiment, step (d) is by performed fluorescence activated cell sorting and the fluorescent activated cell sorting profile of the clonal population selected at step (d) indicates a narrow distribution around the mean, thereby indicating a clonal population stably expressing the target polypeptide.

In another embodiment, step (d) is performed 7-28 days after step (c), or alternatively, after 7 days, or alternatively after 10 days, or alternatively, after 15 days, or alternatively, after 20 days, or alternatively, after 25 days, or yet further after 28 days, after step (c).

In certain embodiments, a high expression level refers to the level of expression of the cell surface marker that is higher than the expression level of the cell surface marker on at least 50, 70, 80, 90, 95 or 99% of the cells analyzed in step (b) and/or step (d).

In another embodiment flow cytometry is combined with a non-fluorescent cell surface marker for use in the methods of the invention.

The methods of the disclosure identify recombinant cells that maintain protein production or expression levels of a target polypeptide at +/- 10%, or alternatively +/- 15%, or alternatively +/- 20%, or alternatively +/- 25%, or alternatively +/- 30%, or alternatively +/- 35%, or alternatively +/- 40%, or yet further at +/- 50%, as compared to protein production taken over a period of time, measured, by example, by 5 or more, or alternatively 10 or more, or alternatively 15 or more, or alternatively 20 or more, or alternatively 25 or more, or alternatively 30 or more, or alternatively 35 or more, or alternatively 40 or alternatively, 45 or more, or alternatively 50 or more, or alternatively 55 or more or alternatively 60 or more cell generations.

Thus, in one aspect, this disclosure provides methods to identify, select and scale up to a clonal population of recombinant eukaryotic cells that stably express a target polypeptide at a high expression level, suitable for large scale, e.g. commercial, production of the target polypeptide.

The methods make use of a recombinant eukaryotic host cell, such as those disclosed herein. The recombinant eukaryotic host cell contains or comprises a recombinant polynucleotide, wherein the recombinant polynucleotide comprises a promoter element; a polynucleotide encoding a cell surface marker polypeptide; a polynucleotide encoding a target polypeptide; and a polynucleotide having the biological activity of an internal ribosome entry site (IRES) polynucleotide, and wherein the IRES polynucleotide is located within the recombinant polynucleotide such that the cell surface marker polynucleotide and the target polynucleotide are transcribed on the same mRNA. In an alternate embodiment, the cell surface marker polynucleotide includes an alternate start codon for translation initiation. Recombinant host cells are bound by an agent which binds directly or indirectly the cell surface marker and such cells are then selected and isolated. The selected and isolated cells are then prepared into one or more clonal populations and cultured for a period of time. The clonal populations are then analyzed by detecting the level of the cell surface marker expression on said clonal population and one or more clonal populations with high expression levels of the cell surface marker are selected. The selected clonal populations with high expression levels of the cell surface marker indicate clonal populations with stable and high expression levels of the target polypeptide. The cells selected by these methods can be further cultured under conditions that enable the production of the target polypeptide that also is present in the host cell. The target polypeptide can be further isolated from the cells or cell culture media as appropriate.

An assembly to perform the methods, discussed in more detail below, also is provided by this disclosure. In one aspect, the assembly comprises i) a recombinant eukaryotic host cell that contains a polynucleotide that produces, on the same mRNA, a polynucleotide encoding a cell surface marker polypeptide and a target polypeptide; ii) an agent that will recognize through direct or indirect binding to the expression product of the polynucleotide encoding the cell surface marker and a means to identify host cells that have agent:marker complexes after being brought into proximity to each other so that the agent can bind the marker if it is present on the recombinant host cell. For example, the assembly may further include a multi well plate for screening concurrent multiple screens or selections, e.g., a 6-well, a 12-well, a 24-well, 48-well, a 96-well or alternatively, a 384-well microtiter plate and therefore, the method of this invention is not intended to be limited to the exemplified 6- or 96-well microtiter culture plates.

The disclosure also provides a eukaryotic host cell containing the recombinant polynucleotide compring: a promoter element; a polynucleotide encoding a cell surface marker polypeptide; a polynucleotide encoding the target polypeptide; and a polynucleotide having the biological activity of an internal ribosome entry site (IRES) polynucleotide wherein the IRES polynucleotide is located within the recombinant polynucleotide such that the polynucleotide encoding the cell surface marker polypeptide and the polynucleotide encoding the target polypeptide are transcribed on the same mRNA. In an alternate embodiment, the polynucleotide encoding the cell surface marker polypeptide further contains an alternate start codon for translation initiation, in addition to or in lieu of, the IRES element. In this embodiment, the polynucleotide encoding the target polypeptide is located downstream from the polynucleotide encoding the marker polypeptide.

### Host Cells

Examples of suitable eukaryotic cells to contain the recombinant polynucleotide include, but are not limited to, the exemplified Chinese Hamster Ovary cell line, including those designated CHO-K1, DG44, DUKX (also called DXB11), and CHO-S (commercially available from Invitrogen) and the hamster cell line BHK-21; the murine cell lines designated NIH3T3, NS0, C127, the simian cell lines COS, Vero; and the human cell lines HeLa, HEK293 (also called 293), PER.C6 (commercially available from Crucell) U-937 and Hep G2. Additional examples include yeast cells, insect cells, plant cells, avian cells, fungal cells and bovine cells. Examples of yeast useful for expression include, but are not limited to Saccharomyces, Schizosaccharomyces, Hansenula, Candida, Torulopsis, Yarrowia, or Pichia. See e.g., U.S. Pat. Nos. 4,812,405; 4,818,700; 4,929,555; 5,736,383; 5,955,349; 5,888,768 and 6,258,559. The eukaryotic cells can be purchased from a commercial vendor such as the American Type Culture Collection (ATCC, Rockville Maryland, USA) or cultured from an isolate using methods known in the art.

In one aspect, the host cells have been pre-screened and selected for stable production of the protein or peptide of interest. Applicants have used chemical selection with methotrexate but other selection methods are known in the art with some examples described in Liu et al. (2000) Anal. Biochem. 280:20-28; Barnes et al. (2003) Biotechnol. Bioeng. 81:631-639; Sautter and Enenkel (2005) Biotechnol. Bioeng. 89:530-538. In an alternate embodiment, the cells have not been preselected for stable production of the target protein or polypeptide.

The eukaryotic host cells of the invention contain a recombinant polynucleotide which can be a recombinant mRNA or cDNA molecule that results in an mRNA molecule from which the target and marker polypeptides separately translated. This is accomplished by use of a polynucleotide that has the biological activity of an internal ribosome entry site (IRES) located on the same polynucleotide encoding the marker and target polypeptides. In one aspect, the IRES element is located upstream from the polynucleotide encoding the target polypeptide and downstream from the polynucleotide encoding the cell surface marker. In another aspect, the IRES element is located upstream from the polynucleotide encoding the cell surface marker and downstream from the polynucleotide encoding the target polypeptide.

In an alternate embodiment, the target polypeptide and the marker polypeptide are encoded on the same mRNA but only one or the other polypeptide is translated from a given mRNA molecule. This is accomplished by use of an alternate (i.e., non-ATG) start codon for translation initiation of the marker polypeptide and the use of an ATG start codon for translation initiation of the target polypeptide. In this embodiment, the polynucleotide encoding the target polypeptide is located downstream from the polynucleotide encoding the marker polypeptide. (See, for example, Figure 7).

To make the recombinant eukaryotic host cell, the recombinant polynucleotide(s) can be directly inserted into the host cell using any suitable gene transfer technique. Alternatively, a vector for insertion of the polynucleotide(s) into the host cell can be used. Suitable vectors include, but are not limited to viral vectors, viral-associated vectors and plasmids. Examples of suitable DNA viral vectors include adenovirus (Ad) or adeno-associated virus (AAV). Adenovirus-based vectors for the delivery of polynucleotides are known in the art and may be obtained commercially or constructed by standard molecular biological methods. Adenoviruses (Ads) are a group of viruses, including over 50 serotypes. See, e.g., International PCT Application No. WO 95/27071. Ads do not require integration into the host cell genome. Recombinant Ad derived vectors, particularly those that reduce the potential for recombination and generation of wild-type virus, have also been constructed. See, International PCT Application Nos. WO 95/00655 and WO 95/11984. In general, recombinant adenoviral vectors derived from adenovirus type 2 (Ad2) and adenovirus type 5 (Ad5). They may also be derived from other non-oncogenic serotypes. See, for example, Horowitz, "Adenoviridae and their Replication" in VIROLOGY, 2d ed., Fields et al. Eds., Raven Press Ltd., New York, 1990.

Other viral vectors for use in the present disclosure include vectors derived from vaccinia, herpesvirus, and retroviruses. In particular, herpesviruses, especially herpes simplex virus (HSV), such as those disclosed in U.S. Pat. No. 5,672,344.

Vectors that contain both a promoter and a cloning site into which a polynucleotide can be operatively linked are known in the art and available from commercial vendors. Such vectors are capable of transcribing RNA in vitro or in vivo, and are commercially available from sources such as Stratagene (La Jolla, CA) and Promega Biotech (Madison, WI). In order to optimize expression and/or in vitro transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

Gene delivery vehicles also include several non-viral vectors, including DNA/liposome complexes, and targeted viral protein-DNA complexes. Liposomes that also comprise a targeting antibody or fragment thereof can be used in the methods of this invention. To enhance delivery to a cell, the nucleic acid or proteins of this invention can be conjugated to antibodies or binding fragments thereof which bind cell surface antigens, e.g., TCR or CD3.

Yet further disclosed are recombinant host cells having any one of the embodiments described herein and further containing a second recombinant polynucleotide encoding a second target protein or polypeptide under the control of the first or a second promoter element.

### IRES

An IRES or a polynucleotide having the biological activity of IRES can be identical to sequences found in nature, such as the picornavirus IRES, or they can be non-naturally or non-native sequences that perform the same function when introduced into a suitable host cell. For example, bi- and poly-cistronic expression vectors containing naturally occurring IRES elements are known in the art and described for example in Mosser et al. (1997) BioTechniques 22(1):150-161; Pestova et al. (1998) Genes Dev. 12:67-83; Chen et al. (2004) J. Immunol. Methods 295:49-56 and International Application No. WO 01/04306, which in turn on page 17, lines 35 to 38 cites several literature references which include, but are not limited to Ramesh et al. (1996) Nucl. Acids Res. 24:2697-2700; Pelletier et al. (1988) Nature 334:320-325; Jan et al. (1989) J. Virol. 63:1651-1660; and Davies et al. (1992) J. Virol. 66:1924-1932. Paragraph [0009] of U.S. Patent Appl. Publ. No.: 2005/0014150 A1 discloses several issued U.S. patents wherein a virally-derived IRES element was used to express foreign gene(s) in linear multi-cistronic mRNAs in mammalian cells, plant cells and generally in eukaryotic cells. U.S. Patent Appl. Publ. No. 2004/0082034 A1 discloses an IRES element active in insect cells. Methods to identify new IRES-like elements also are described in U.S. Patent No. 6,833,254. Because the DNA encoding the marker polypeptide and the DNA encoding the target polypeptide are transcribed on the same mRNA, the expression level of the marker polypeptide can predict the relative levels of the target polypeptide on a per cell basis.

In the exemplified host cells described below, the IRES was purchased from Clontech Laboratories (pIRES Vector) and Sequence of IRES is provided on the vendor's web site. This IRES used has been mutated by the vendor (ATGs 11 and 12 deleted) for weaker translation efficiency (as described in pIRES Vector Information, Clontech Laboratories, Inc. (2005) Protocol No. PT3266-5, Version No. PR59976 and references therein).

Also intended by the use of the term IRES or a polynucleotide having the biological activity of IRES are cellular sequences similar to that disclosed in U.S. Patent No. 6,653,132. The patent discloses a sequence element (designated SP163) composed of sequences derived from the 5'-UTR of VEGF (Vascular Endothelial Growth Factor gene), which, was presumably generated through a previously unknown mode of alternative splicing. The patentees report that an advantage of SP163 is that it is a natural cellular IRES element with a superior performance as a translation stimulator and as a mediator of cap-independent translation relative to known cellular IRES elements and that these functions are maintained under stress conditions.

Further intended by the use of the term IRES or a polynucleotide having the biological activity of IRES are other non-naturally occurring sequences that function as IRES elements which are described, for example, in U.S. Patent Appl. Publ. No.: 2005/0059004 A1.

Operatively linked to the IRES element are polynucleotides encoding the cell surface marker and target polypeptides. "Operatively linked" means positioned in an arrangement that allows them to function. Other elements which may be operable linked to the above assembly include but are not limited to a promoter, an enhancer, a termination sequence and a polyadenylation sequence. Construction and use of such sequences are known in the art and are combined with IRES elements and protein sequences using recombinant methods.

### Alternate Start Codons

In the invention, instead of an IRES, an alternate start codon is located within the DNA encoding the marker polypeptide in such a way that translation of the marker polypeptide is less efficient than that of the target polypeptide. To achieve decreased translation efficiency, the ATG start codon of the marker polypeptide is changed to an alternate start codon, examples of which include but are not limited to: CTG, GTG, TTG, ATT, ATA, ACG.

Thus, when using an alternate start codon expression of a reporter (or marker) polypeptide or protein can be attenuated relative to that of a co-expressed target polypeptide of protein by altering the start codon used for translation initiation of the marker polypeptide or protein. In addition to alteration of the start codon, the DNA encoding the marker polypeptide is modified at all internal ATG triplets to prevent internal initiation of translation. In one embodiment, the marker polypeptide has a short amino acid sequence (<200 amino acids) with few (<10) ATG triplets. In one embodiment, all of the internal ATG triplets of the marker polypeptide are not in frame for the encoded polypeptide sequence. In one embodiment, each internal ATG triplet that is in frame for the encoded polypeptide sequence is changed to a TTG, which encodes the amino acid leucine..

For translation of the mRNA encoding both the marker polypeptide and the target polypeptide, ribosomes begin scanning at the 5' cap structure of the mRNA with the majority scanning past the alternate start codon (for example, GUG) and instead initiating translation at the downstream AUG start codon (for example, see Figure 7). However, translation initiation can occur at the alternate start codon with very low frequency so that a low level of the reporter polypeptide is expressed (for example, see figure 8). In this aspect of the invention, the cell surface marker polynucleotide and the target polynucleotide are transcribed on the same mRNA, so that the expression level of the reporter polypeptide can be used to predict the relative expression level of the target polypeptide on a per cell basis.

Thus, in this aspect of the invention, clonal populations are screened via detection of a marker polypeptide that is expressed using an alternate start codon. Clonal populations with high expression levels of the marker polypeptide are selected as clonal populations that will have high expression of the co-expressed target polypeptide.

In a further aspect of this invention, an alternate start codon is utilized for translation initiation of the marker polypeptide in order to obtain a lower level of marker polypeptide expression relative to that of the target polypeptide. In this embodiment, the polynucleotide encoding the target polypeptide is located downstream from the polynucleotide encoding the marker polypeptide (see, for example, Figure 7). In this aspect of the invention, the use of an alternate start codon to express a marker polypeptide that is, in turn, detected on cells in a high throughput clone screen, such as set forth in the methods of the invention, is an intended use of an alternate start codon.

### Promoters

Promoters are sequences which drive transcription of the marker or target protein. They must be selected for use in the particular host cell, i.e., mammalian, insect or plant. Viral or mammalian promoters will function in mammalian cells. The promoters can be constitutive or inducible, examples of which are known and described in the art and can be located upstream to the polynucleotide encoding the cell surface marker polypeptide and upstream from the polynucleotide encoding the target polypeptide. In another aspect, the promoter is operatively linked to the polynucleotide encoding the cell surface marker polypeptide. In an alternate embodiment, the promoter is operatively linked to the polynucleotide encoding the target polypeptide.

Exemplary promoters include but are not limited to viral, mammalian or yeast promoters that provide for higher levels of expression, e.g., beta-actin, a mammalian CMV promoter, a yeast alcohol oxidase, a phosphoglycerokinase promoter, a lactose inducible promoters, a galactosidase promoter, an adeno-associated viral promoter, a baculovirus promoter, a poxvirus promoter, a retroviral promoter, an adenovirus promoters, an SV40 promoter, a TK (thymidine kinase) promoter, a 7.5K or H5R poxvirus promoter, an adenovirus type 2 MPC late promoter, an alpha-antrypsin promoter, a factor IX promoter, an immunoglobulin promoter, a CFTR surfactant promoter, an albumin promoter or a transferrin promoter.

Further disclosed are recombinant host cells having any one of the embodiments described above and further containing, on the recombinant polynucleotide, a polynucleotide that increases or enhances transcription, i.e., an enhancer element.

### Cell Surface Markers

As used herein, the term "cell surface marker polypeptide" or "marker polypeptide" is used broadly and is intended to include any polypeptide expressed by the cell that will serve to identify and optionally select cells. Examples of cell surface marker polypeptides include, but are not limited to cell surface receptor polypeptides, e.g., CD2, CD20, CD52 or CD59. In a separate embodiment, the cell surface marker polypeptide is not a CD4 polypeptide or a polynucleotide encoding a CD4 polypeptide. Additional exemplary cell surface marker polypeptides are identified at the web address <ebioscience.com/ebioscience/whatsnew/humancdchart.htm>, as of the filing date of this application.

In another aspect, the polynucleotide encoding the cell surface marker polypeptide contained in the host cell is a polynucleotide encoding a polypeptide that is exogenous to the eukaryotic host cell. In another aspect, a polynucleotide encoding the cell surface marker polypeptide is endogenously present in the host cell but expressed at low levels in the untransfected host cell.

In a particular aspect of this disclosure, the polynucleotide encodes CD20. Sequences that encode CD20 markers are known in the art and can be found under the following GenBank Accession numbers: NM_152866; NM_152867; NM_021950; NM 007641 and NM_001009388. In some aspects of this invention, it may be desirable to use non-human genes, the polynucleotide sequences of which are known in the art. See for example, Genbank Accession numbers NM_007641, NM_001009388, and XM_542548.

The cell surface marker polypeptide is used to identify those cells that stably express the target polypeptide at high levels and are therefore candidate clones for larger scale production.

### Target Polypeptides

The target polypeptide can be any protein or polypeptide that can be produced in host cells and in the aspects exemplified herein, the target polypeptide is selected because of its potential as a therapeutic agent or drug, e.g., an antibody, an antibody fragment, or enzyme. However, the assemblies and their uses are not limited for the selection and scale-up of therapeutic proteins. For example, diagnostic or proteins for use in the environment can be identified for scale-up using the methods and assemblies disclosed herein.

In one aspect, the target polypeptide is thyroid stimulating hormone. In alternative aspects, the target polypeptide comprises a lysosomal storage disorder (LSD) protein or polypeptide, which may include, but not be limited to those identified in the below table.

Examples of LSDs and Corresponding Defective or Deficient Enzymes

| **Lysosomal storage disorder** | **Defective or Deficient enzyme (Lysosomal hydrolase)** |
|---|---|
| Fabry | α-Galactosidase A |
| Farber | Acid ceramidase |
| Fucosidosis | Acid α -L-fucosidase |
| Gaucher types 1, 2 and 3 | Acid β-glucocerebrosidase (GCR) |
| G_{MI} gangliosidosis | Acid β-galactosidase |
| Hunter | Iduronate-2-sulfatase |
| Hunter-Scheie | α -L-Iduronidase |
| Krabbe | Galactocerebrosidase |
| α-Mannosidosis | Acid α-mannosidase |
| β-Mannosidosis | Acid β-mannosidase |
| Marateaux-Lamy | Arylsulfatase B |
| Metachromatic leukodystrophy | Arylsulfatase A |
| Morquio A | *N*-Acetylgalactosamine-6-sulfate sulfatase |
| Morquio B | Acid β -galactosidase |
| Niemann-Pick | Acid sphingomyelinase |
| Pompe | Acid α —glucosidase |
| Sandhoff | β -Hexosaminidase B |
| Sanfilippo A | Heparan *N*-sulfatase |
| Sanfilippo B | α -*N*-Acetylglucosaminidase |
| Sanfilippo C | Acetyl-CoA: α -glucosaminide N-acetyl transferase |
| Sanfilippo D | *N*-Acetylglucosamine-6-sulfate sulfatase |
| Schindler-Kanzaki | α -*N*-Acetylgalactosaminidase |
| Sialidosis | Sialidase |
| Sly | β Glucuronidase |
| Tay-Sachs | β -Hexosaminidase A |

Lysosomal storage disorders are a class of genetic diseases, comprising over forty disorders that relate to a deficiency in lysosomal hydrolase activity. The lysosome serves as a major degradative compartment of the cell and contains multiple enzymes necessary to carry out this function. A hallmark feature of LSDs is the abnormal accumulation of metabolites in the lysosomes which leads to the formation of large numbers of distended lysosomes. Accordingly, an LSD may be treated with the administration of an enzyme replacement therapeutic corresponding to the defective or deficient lysosomal hydrolase correlated with the particular LSD.

In one aspect, the target polypeptide is thyroid stimulating hormone. In alternative aspects, the target polypeptide comprises a lysosomal storage disorder (LSD) protein or polypeptide, which may include, but not be limited to those identified in the above table.

In one aspect, an additional polypeptide is transcribed and translated from a separate recombinant polynucleotide and combined into a functional protein in the host cell. This recombinant polynucleotide does not require the IRES element or marker protein although in one aspect, it may be present.

One of skill in the art will understand that the polynucleotides and host cells containing them must be produced prior to practicing the disclosed methods. Depending on the particular components, one skilled in the art can purchase the components and/or elements of the components from commercial vendors.

### Detection of the cell surface markers

After insertion of the recombinant polynucleotide, the host cell is cultured under conditions that facilitate expression of the cell surface marker on the host cell. Any method known in the art useful for detecting the cell surface marker may be used in connection with the methods of the invention. For example, an antibody or other cell surface marker-specific binding agent is then contacted directly or indirectly with the cell under conditions that favor binding of antibody to the marker and therefore the host cell. The selection of the binding agent or antibody is determined by: 1) its ability to selectively bind the cell surface marker polypeptide that is expressed on the host cell; and 2) its ability to be labeled with a detectable label or bind to a detectable label, for example, for use in flow cytometry. Fluorescence activated cell sorting (FACS), also called flow cytometry, is used to sort individual cells on the basis of optical properties, including fluorescence. It is used to screen large populations of cells in a relatively short period of time. Other screening methods include MACs, as described in Gaines (1999) Biotechniques 26(4):683-688.

In one aspect, the agent that binds the cell surface marker is an antibody. The antibody can be a polyclonal or a monoclonal antibody, or it can be fragments of the polyclonal or monoclonal antibody. They can be chimeric, humanized, bi-specific, bi-functional or totally human. Any functional fragment or derivative of an antibody can be used such as Fab, Fab', Fab2, Fab'2, single chain variable regions and variations of the same.

In an alternate embodiment, a first agent can be a protein or peptide that binds to the marker polypeptide which first agent also in turn binds to a second agent that is capable of being detectably labeled. It is intended, although not always explicitly stated that "indirect" binding to the marker includes the use of any number of intermediate partners. In this embodiment, attachment of the first labeling moiety to the candidate agents will be done as is generally appreciated by those in the art, and may include techniques outlined above for the incorporation of fluorescent, enzymatic, colormetric, or other detectable labels.

In one embodiment, the agent binds directly to the cell surface marker and comprises a fluorescent label. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade Blue.TM., and Texas Red. Other suitable optical dyes are described in the 1996 Molecular Probes Handbook by Richard P. Haugland.

In another aspect, the fluorescent label is functionalized to facilitate covalent attachment to the agent. Suitable functional groups, including, but not are limited to, isothiocyanate groups, amino groups, haloacetyl groups, maleimides, succinimidyl esters, and sulfonyl halides, all of which may be used to attach the fluorescent label to a second molecule. The choice of the functional group of the fluorescent label will depend on the site of attachment to either a linker, the agent, the marker, or the second labeling agent.

Attachment of the fluorescent label may be either direct or via a linker to the antibody and/or agent. In one aspect, the linker is a relatively short coupling moiety, that generally is used to attach molecules. In this embodiment, attachment of the first labeling moiety to the candidate agents will be done as is generally appreciated by those in the art, and may include techniques outlined above for the incorporation of fluorescent labels.

Materials and techniques for design and construction of labeled antibodies and other agents for use in cytometry are known in the art and described for example, in Bailey et al. (2002) Biotech. Bioeng. 80(6):670-676; Carroll and Al-Rubeai (2004) Expt. Opin. Biol. Therapy 4:1821-1829; Yoshikawa et al. (2001) Biotech. Bioeng. 74:435-442; Meng et al. (2000) Gene 242:201-207; Borth et al. (2001) Biotechnol. Bioeng. 71 (4):266-273; Zeyda et al. (1999) Biotechnol. Prog. 15:953-957; Klucher et al. (1997) Nucleic Acids Res. 25(23):4853-4860; and Brezinsky et al. (2003) J. Immunol. Methods 277:141-155.

Suitable binding pairs for use in indirectly linking the label to the agent (which in turn, binds the marker) include, but are not limited to, antigens/antibodies, including digoxigenin/antibody, dinitrophenyl (DNP)/anti-DNP, dansyl-X/anti-dansyl, fluorescein/anti-fluorescein, lucifer yellow/anti-lucifer yellow, rhodamine/anti-rhodamine; and biotin/avidin (or biotin/strepavidin). The binding pairs should have high affinities for each other, sufficient to withstand the shear forces during FACS sorting or other detection system used in connection with the invention..

Thus, in some aspects, first labeling moieties (when second labeling moieties are used), include, but are not limited to, haptens such as biotin. Biotinylation of target molecules is well known, for example, a large number of biotinylation agents are known, including amine-reactive and thiol-reactive agents, for the biotinylation of proteins, nucleic acids, carbohydrates, carboxylic acids. Similarly, a large number of haptenylation reagents are also known.

The antibodies used for the assembly can be produced in cell culture, in phage, or in various animals, including but not limited to cows, rabbits, goats, mice, rats, hamsters, guinea pigs, sheep, dogs, cats, monkeys, chimpanzees, apes, etc., so long as the fragment or derivative retains specificity of binding for the protein or fragment. Antibodies can be tested for specificity of binding by comparing binding to appropriate antigen to binding to irrelevant antigen or antigen mixture under a given set of conditions.

In embodiments in which the antibody or agent against the cell surface marker is not directly labeled, the antibody or agent preferably also contains and retains the ability to bind a secondary agent which is detectable after binding to the host cell via the cell surface marker polypeptide. The labels themselves must be capable of detection, e.g., by flow cytometry using fluorescent labels, or MACS which are suitable exemplary labels for use in the invention.

In a particular embodiment, when the cell surface marker is CD20, the antibody of the assembly is an anti-CD20 antibody. "Anti-CD20 antibody" refers to an antibody that specifically recognizes and binds CD20 polypeptide or protein. Anti-CD20.antibodies can be generated by methods well known in the art. See for example, Harlow and Lane, eds. (1988) supra and Freshney, ed. (1987) supra. Additionally, several anti-CD20 antibodies are commercially available from vendors such as BD Pharmigen; Beckman Coulter, Inc. (Fullerton, CA, numerous clones including Catalog No. 6604106 Clone H299 (B1); Isotype IgG2a and Catalog No. IM1565 Clone L26, Isotype IgG2a); Invitrogen (Carlsbad, CA, Clone: BH-20, Isotype: IgG2a and Clone: B-H20, Isotype: IgG2a); BioLegend (San Diego, CA, Catalog No. 302301, Clone: 2H7, Isotype: IgG2b, ê); EMD Biosciences, Inc., CALBIOCHEM® Brand (San Diego, CA, Catalog No. 217670 Clone 2H7, Isotype: IgG2b); and Anaspec (San Jose, CA, Catalog No. 29587).

To practice the method, at least one recombinant eukaryotic host cell as described herein is contacted with an agent that recognizes and directly or indirectly binds the cell surface marker, if present, on the surface of the host cell. The contacting is performed under conditions that that favor or are suitable for specific binding (directly or indirectly) of the agent or antibody with the marker and then selecting any host cell that is directly or indirectly bound to the agent or antibody that is bound to the cell surface marker polypeptide. The method includes selecting and isolating one or more host cells that is bound to the agent and preparing clonal populations using such selected cells. Preparation of a clonal population can be performed by any method known in the art. For example, in one embodiment, the selected cells may be plated into 96-well (or other size) plates at a density of one cell per well and permitted to grow for a period of time (e.g., typically 7-28 days) which permits the single cell to grow into a multi-cell colony of daughter cells (i.e., a clonal population). The method next comprises analyzing one or more of the clonal populations by detecting the level of the cell surface marker expression on said clonal population and selecting one or more clonal populations with a high expression level of the cell surface marker, thereby selecting one or more clonal populations stably expressing the target polypeptide. In certain embodiments, the clonal population is cultured for 7-28 days after plating at a single cell density before the clonal populations are analyzed. The method further includes contacting the clonal population with a detectable agent that recognizes and directly or indirectly binds the cell surface marker, if present, on the surface of the host cell under conditions that favor binding of the agent with the marker; and selecting one or more host cells that are directly or indirectly bound to the agent antibody that is bound to the foreign cell surface marker polypeptide. These cells so selected also can be isolated and cultured. The high expression level clonal population cells are termed "producer cells."

The producer cells can be used to produce a target polypeptide that can be further isolated from the cell and/or cell culture.

Selection of the producer cell or cells can be performed as exemplified herein using a fluorescent activated cell sorter or a magnetic activated cell sorter (MACS), or by any method known in the art.

Also provided is the assembly to perform the methods disclosed herein. In these aspects, the assembly contains at least one eukaryotic host cell as described herein, an agent that directly or indirectly binds the cell surface marker polypeptide; and a means for detecting the agent when it is directly or indirectly bound to the cell surface marker polypeptide. Suitable host cells, agents and detection and selection means are described above, and also are known in the art.

The following experimental examples are intended to illustrate, not limit the invention.

### EXAMPLES

### Materials and Methods

**Construction of DNA Expression Vectors.** For polypeptides co-expressed with CD20 [soluble receptor, antibody heavy chain (Hc), and thyroid stimulating hormone (TSH) (β-subunit], the open reading frames were inserted into a mammalian expression vector downstream of the hamster β-actin promoter and upstream of the IRES, which was followed by a human CD20 cDNA and SV40 Poly(A). In the IRES sequence (pIRES, Clontech Laboratories, Mountain View, CA), AUGs 11 and 12 were deleted, decreasing the translation initiation efficiency of the site (Davies and Kaufman (1992) J. Virol. 66:1924-1932 and pIRES Vector Information, Clontech Laboratories, Inc. (2005) Protocol No. PT3266-5, Version No. PR59976). For polypeptides not co-expressed with CD20 [antibody light chain (Lc) and TSH α subunit], the open reading frames were inserted into a mammalian expression vector downstream of the hamster β-actin promoter and upstream of the SV40 Poly(A). All vector backbones also contained a DHFR expression cassette to enable MTX selection in nucleotide-deficient medium.

**Cell Culture and Transfection**. Unless otherwise noted, all media and supplements were from Invitrogen (Carlsbad, CA). The CHO DXB11 cell line (Urlaub and Chasin (1980) Proc. Natl. Acad. Sci. USA 77 (7):4216-4220) was adapted to animal derived component (ADC)-free growth conditions using methods and materials known in the art. The CHO SF-DG44 cell line was obtained from Invitrogen. Cells were grown in suspension using vented cap shake flasks (Coming, Corning, NY) in a Multitron incubator (Infors HT, Bottmingen, Switzerland) at 37°C, 5% CO₂, 70% humidity, and shaking at 125 rpm. Growth medium was CD DG44 supplemented with 4mM L-glutamine and 0.01% Pluronic F-68. Cells were transfected by electroporation using a GenePulser Xcell™ (BioRad Laboratories, Hercules, CA) with either a single plasmid (soluble receptor) or with equimolar amounts of two plasmids (antibody Lc + antibody Hc; TSH α + TSH β). All plasmid DNAs were linearized and EtOH precipitated prior to transfection. After a recovery period of 48-72 hours, transfected cells were reseeded in nucleotide-deficient CD CHO medium supplemented with 4 mM L-glutamine. Stable pools were subsequently subjected to either a single MTX (Calbiochem/EMD Biosciences, San Diego, CA) selection step (100 nM) or a two-step MTX selection process (20 nM/100 nM or 20 nM/200 nM). Immediately after sorting, cells were seeded into 96-well plates at 1 cell/well in MTX-free medium, and wells were visually inspected 4 to 7 days post-sort for identification of single cell clones. Clones were maintained in MTX-free CD CHO from this point on.

**Flow Cytometry Sorting and Clone Screening**. Seven to ten days prior to sorting, stably transfected pools were seeded in MTX-free CD CHO medium supplemented with 4mM L-glutamine, and were reseeded in this medium as needed until the day of sorting. All steps in the sort and recovery process utilized sterile reagents and were carried out under sterile conditions. All washes were performed in cold phosphate buffered saline PBS (Invitrogen). In preparation for sorting, cells were incubated with FITC-conjugated anti-human CD20 antibody (BD Pharmingen, San Diego, CA) for 30 minutes on ice, with washes before and after antibody incubation. Cells were resuspended in cold PBS at a concentration of 1 X 10⁶ viable cells/ml and sorted on a FACStar^{PLUS} flow cytometer (BD Biosciences, San Jose, CA) using an argon laser emitting at 488nm and detecting FITC emission with a 530/30 bandpass filter. The sort gate was a combination of the live cell gate (from FSC-H vs. SSC-H dot plot) and the events in the top 0.1-1.0% fluorescence (from FL-1 histogram). For clone screening at the 6-well plate and shake flask stages, 1 X 10⁶ to 2 X 10⁶ viable cells were harvested at time points specified. Cells were prepared exactly as described above for sorting, resuspended in cold PBS at approximately 1 X 10⁶ viable cells/ml, and analyzed on a FACSCalibur flow cytometer (BD Biosciences) using an argon laser emitting at 488 nm and detecting FITC emission with a 530/30 bandpass filter. For clone screening at the 96-well plate stage, approximately 50% of the cells in a 70-90% confluent well were transferred to a single well of a 96-well polypropylene V-bottom plate (Corning). Cells were washed, incubated with PE-conjugated anti-human CD20 antibody (BD Pharmingen) for 30 minutes on ice, washed, and resuspended in 100 µl cold PBS per well. Cells were analyzed on a FACSArray™ Bioanalyzer (BD Biosciences) using an argon laser emitting at 532 nm and detecting PE emission with a 585/42 bandpass filter. For all flow cytometry procedures, CellQuest and Cell QuestPro software programs (BD Biosciences) were used to analyze data. Histograms shown were generated using Cell Quest Pro or FlowJo (Tree Star Inc., Ashland, OR) software programs. For FACS clone screening, each result is a measure of the geometric mean fluorescence intensity (GMFI), quantified in relative fluorescence units (RFU).

**Analysis of Protein Titers**. For 6-well plate analysis, cells were seeded into wells as described in the description of the figures, and conditioned media was harvested from the wells at either day 3 or day 7 post-seeding, as specified. For unfed batch culture analysis, cells were seeded in 125 ml shake flasks in 20 ml of CD CHO/4 mM L-glutamine and incubated in a Multitron incubator as described above. Cells producing soluble receptor or antibody proteins (as target polypeptides) were seeded at 3 X 10⁵ viable cells/ml and conditioned media was harvested at day 14. Cells producing TSH (as the target polypeptide) were seeded at 1 X 10⁶ viable cells/ml and conditioned media was harvested at 24 hours. Conditioned media samples were centrifuged to remove cells and debris, then stored at -80°C. For determination of soluble receptor (an F_{c} fusion) and antibody (IgG₄) protein titers, conditioned media was analyzed by Protein A HPLC. For TSH samples, total cell numbers were determined for calculation of per cell productivity and TSH titers were determined by ELISA.

### Results

**Accurate Prediction of Clonal Therapeutic Protein Titers By the CD20-Based FACS Screen**. A reporter-based FACS process was initially developed to sort cells with high target protein expression levels. The gene encoding the protein of interest (target protein) was co-expressed from a mammalian expression vector with the cell surface reporter protein CD20 (cell surface marker polypeptide), not normally expressed on CHO cells (Figure 1). In this system, CD20 protein expression levels were used to accurately predict the relative expression levels of the therapeutic target protein on a per cell basis. After stable transfection of CHO cells, MTX-selected pools were assayed for cell surface CD20 expression by flow cytometry. Pools with high fluorescence levels were sorted to collect individual cells with high CD20 expression levels.

After sorting, cells were seeded into 96-well plates to obtain single cell clones (for preparation of clonal population). Typically, clones in 96-well plates were screened by analysis of conditioned media from wells at approximately 80-90% confluence. Based on this screen, the top 50-100 clones were selected and scaled up and subsequently screened by analysis of conditioned media from 6-well plates to predict which clones would have high protein titers at larger scale. Because one aim of the initial study was to reduce the turn around time from seeding 6-well plates to identifying the stable and high expression level clones, an early analytical time point, such as day 3 or 4, was more desirable than a terminal time point of 10-14 days. Surprisingly, during early development of CHO clones expressing a recombinant soluble receptor, CD20-based FACS analysis at 3 days after seeding 6-well plates was observed to be very predictive of the soluble receptor protein titer at a later stage (125-ml shake flask). In fact, this correlation was better than that between the 6-well plate conditioned media protein titer and the shake flask protein titer (Figure 2). A similar correlation was observed for CHO clones expressing an IgG₄. In this case, even a later time point (day 7) for conditioned media analysis of the 6-well plates was unable to accurately predict the clones with high expression levels, whereas the day 7 FACS analysis successfully did so (Figure 3). This figure also demonstrates the utility of the FACS method when the target polypeptide was expressed as two polypeptides, only one of which was co-expressed with CD20.

**Quantitative and Qualitative Assessment of Clones By the 96-Well Plate FACS Screen**. Since the CD20-based FACS screen was more predictive than conditioned media analysis at the 6-well plate stage, it was hypothesized that this FACS screen would also have value at the 96-well plate stage. Implementation earlier in the development process would streamline efforts by allowing one to focus only on the clones with stable and high expression levels, without spending time on the scale up and analysis of undesirable clones (i.e., those with unstable or low expression level, or non-clonal populations). To evaluate the FACS screen at the 96-well plate stage, 34 CHO clones expressing the recombinant soluble receptor were screened for CD20 expression at both the 96-well and 6-well plate stages. As shown in Figure 4A, the 96-well and 6-well FACS screens produced the same rank order of clones with respect to CD20 expression level, measured in relative fluorescence units (RFU). In Figure 4B, representative FACS profiles show the relationship of CD20-based fluorescence intensity of cells in a 96-well plate to the soluble receptor protein titer in conditioned media from a 125-ml shake flask seeded approximately three weeks after the 96-well plate screen.

In addition to a quantitative screen at the 96-well plate stage, the FACS method enabled qualitative evaluation of clones to further streamline the cell line development process. At the 96-well plate stage, the FACS profile was used to identify undesirable clones, even those with a favorable quantitative assessment, by visualizing any heterogeneity that may be associated with unstable populations. In one example, for cells expressing an IgG₄ antibody, qualitative differences between wells with the same CD20-based fluorescence were observed (Figure 4C). In this 96-well plate screen, the CD20-based fluorescence intensity of wells 4G2 and 7G4 were 127.5 and 127.1 RFU, respectively. While cells from well 7G4 were expanded based on the narrow distribution of the cell population [around the mean], those in well 4G2 were eliminated from further development, as this population had a greater degree of heterogeneity, indicating clone instability or perhaps a non-clonal population. Thus, the screen of this invention can be used early in the cell line development process to select appropriate clones for producer cells based on both quantitative and qualitative attributes.

**Elimination of Unstable Clones Early in the Development Timeline by the Screen of this Invention**. There was occasionally an analysis for which a small percentage of clones had lower unfed batch shake flask titers than expected based on the 96-well screen of this invention. Such clones may have unstable protein expression, with the decline in expression detectable during scale up after the 96-well plate screen (Bames et al. (2003) Biotech. Bioeng. 81:631-639 and Kim et al. (1998) Biotech. Bioeng. 60:679-688). Rescreening these clones by FACS at the shake flask stage did, in fact, show a decrease in CD20-based fluorescence consistent with the lower therapeutic protein production levels; a representative example is shown in Figure 5. For CHO clones producing TSH, the 96-well FACS screen accurately ranked clones according to specific productivity in shake flask cultures, with two exceptions, clones 11B3 and 1E7. For these two clones having lower productivity than that predicted by the 96-well screen, the shake flask FACS analysis showed a decrease in CD20-based fluorescence that correlated with the lower protein production levels, indicating instability of transgene expression.

Therefore, the screen of this invention can be used not only to successfully identify high production clones (i.e., high expression level clonal populations) at the multi-well plate stage, but also to monitor the top expressing clones after scale up to promptly identify (and eliminate) any with unstable protein expression. To demonstrate this, clones selected as high producers by the 96-well screen were scaled up to shake flasks and seeded in unfed batch cultures to determine IgG titers. The time from the 96-well plate screen to seeding the initial unfed batch culture was 10 days, with the IgG titer evaluated after 14 days of batch culture. Cells were continuously passaged for an additional 6 weeks, with unfed batch cultures seeded every 3 weeks. These unfed batch cultures were evaluated by FACS at day 3, and the IgG titers were determined at day 14. As shown in Figure 6, clone 14A10 showed a decline in CD20-based fluorescence compared to that observed in the 96-well plate screen within the first 3 weeks of scale up. This decrease in CD20 expression was paralleled by a decrease in IgG titer, showing that the screen during clone scale up was an accurate indicator of clone instability. In comparison, clone 29H6 maintained a stable IgG titer, mirrored by stable CD20-based fluorescence, over the same time frame. These data illustrate the utility of the screen of this invention for eliminating unstable clones at any point in the cell line development timeline. Here, one main advantage of the screen of this invention over traditional media analysis is early time point (day 3) analysis coupled with immediate visualization of results, rather than late time point (day 14) analysis with subsequent analytical data processing to determine titers.

### Discussion

The use of flow cytometry to isolate cells expressing desired levels of a recombinant gene of interest has been described (Liu et al. (2000) Anal. Biochem. 280:20-28; Klucher et al. (1997) Nucleic Acids Res. 25(23):4858-4860; Mosser et al. (1997) BioTechniques 22(1):150-161; Gaines et al. (1999) BioTechniques 26(4):683-688; Choe et al. (2005) Nucleic Acids Res. 33:1-7 and Zeyda et al. (1999) Biotechnol. Prog. 15:953-957). More specifically, for isolation of CHO cells secreting high levels of proteins, flow cytometry has emerged as useful tool to increase the efficiency of cell line development processes. In some cases, CHO cells producing proteins of interest have been isolated via direct detection of the protein of interest either during its association with the extracellular matrix during secretion (Brezinsky et al. (2003) J. Immunol. Methods 277:141-155) or via an affinity matrix protein capture technology (Borth et al. (2001) Biotechnol. Bioeng. 71(4):266-273). However, direct detection is limited by the availability of an antibody specific for the therapeutic protein being expressed, and for many recombinant therapeutic proteins, a production cell line is developed prior to having such an antibody available. FACS-based detection of a reporter protein, such as GFP (Bailey et al. (2002) Biotechnol. Bioeng. 80(6):670-676) or a mutant enzyme (Sautter and Enenkel (2005) Biotech. Bioeng. 89:530-538 and Chen (2004) J. Immunol. Meth. 295:49-56), co-expressed with the protein of interest has also been reported for CHO cells. For both the direct and indirect detection procedures, the focus has been on sorting populations of cells to isolate high producing clones. The previous studies describe the enrichment of heterogeneous pools for high producing cells (subpopulations or single clones) or the enrichment of a clonal population for subclones with potentially higher protein expression. While these prior methods have helped improve the efficiency of isolating the desired cells from transfected pools, accurate hight throughput screening of the resulting clones remained an area for improvement.

To this end, this invention provides a reporter-based, FACS clone screen to rapidly identify the stable and high expression level clones at the 96-well plate stage of cell line development. This screen accurately identifies clones based on both quantitative and qualitative attributes, allowing time and resources to focus only on the best clones for further development. This screen is more effective than traditional clone screening methods in two ways. First, it is a better predictor, at an early stage, of the clones that will have high protein titers later in the development timeline. Second, it enables visual assessment of clone quality, a feature that is not possible when evaluating conditioned medium for protein titer. Unstable clones can be easily identified in the FACS screen either as a population with a large degree of heterogeneity in its fluorescence intensity or as two discrete populations. Two distinct populations could also be indicative of a non-clonal population. While this 96-well plating procedure has a high likelihood of generating less than or equal to one (1) cell/well, the possibility still exists for a well to contain a population derived from more than one cell (i.e., a non-clonal population). Using the FACS screen of this invention, clones with different expression profiles that were derived from a single well can be readily distinguished, preventing further effort from being expended on their expansion and continued evaluation.

In addition to being a more effective clone screen, the method of the invention provides other benefits that are instrumental to developing production cell lines for a variety of proteins or target polypeptides. First, because this system relies on the detection of a co-expressed reporter protein, it removes the need for an antibody specific for the target polypeptide. This enables easy implementation of the method of the invention into any new cell line development process at the earliest stage of development. In addition, the CD20 reporter (marker polypeptide) is expressed on the cell surface so that clone differences in the degree of protein retention and/or rate of dissociation from the extracellular matrix are not a factor in the FACS detection and readout. Interestingly, when comparing the method of this invention to that in which the target protein (an IgG) is directly detected by an antibody (either anti-Fc or anti-IgG), this invention more accurately predicted which clones would produce high, medium, or low levels of the protein (target polypeptide). This indicates that, at least for some target proteins, direct detection of the secreted protein may generate a higher percentage of false positive or false negative results, in turn not identifying the clones with stable and high expression level production of the target polypeptide. For example, if a cell produced high levels of a target protein of interest but the protein did not remain associated with the extracellular matrix long enough for detection by an antibody, the cell would be overlooked in the direct detection screen due to a low readout.

Second, the method of the invention performs equally well for target polypeptides expressed either as a single polypeptide or as two polypeptides encoded by separate open reading frames on different expression plasmids. For cells expressing either an antibody or thyroid stimulating hormone, the CD20 reporter protein (marker polypeptide) was co-expressed with only the heavy chain or the β subunit, respectively and was used to successfully identify the clones producing high levels of fully assembled proteins. For antibody production, co-expression of CD20 with the heavy chain provided an added advantage. Recent reports demonstrate that maximal heavy chain expression is necessary for higher production of fully assembled antibody (Schlatter et al. (2005) Biotechnol. Prog. 21:122-133 and Jiang et al. (2006) Biotechnol. Prog. 22:313-318). Therefore, linking the per cell CD20 expression levels to heavy chain expression levels enabled identification of clones with high heavy chain and, in turn, high expression of assembled antibody.

Third, the CD20 based FACS method of the invention provided a rapid, qualitative determination of clone stability during expansion from 96-well plates to shake flasks. This served as a second tier screen of clones that are scaled up based on the initial 96-well screen results. The ability to screen cells 3 days after seeding a batch culture, at any point in the scale up process, generated data more quickly than traditional conditioned media analysis from a terminal batch culture. Furthermore, identifying clone instability at any point during early clone expansion prevented unnecessary effort from being spent on the continued development of undesirable clones. Finally, in addition to its utility with CHO cells, the method of the invention is applicable to other cell types that can be used to express proteins of interest. Likewise, alternative cell surface reporter proteins (i.e., marker polypeptides) could be used, instead of CD20, for co-expression with the protein of interest (i.e., the target polypeptide).

This method provides substantial benefit for cell line development in its ability to effectively screen clones at the 96-well plate stage for those with stable, high expression of proteins of interest. For multiple cell line development efforts, the 96-well FACS screen successfully identified the clones that would have high protein titer upon scale up with higher accuracy than titer measurements in the 96-well conditioned media. Furthermore, the 96-well plate format allowed for high throughput processing of clones. Using a plate-based flow cytometer, over 1,000 clones can be analyzed in a single day, significantly reducing the time and effort required for clone screening. An additional advantage of this method was that declines in CD20-based fluorescence during clone scale up correlated directly with decreases in therapeutic protein production observed for unstable clones, allowing for rapid identification and elimination of such clones. Using this method, new therapeutic cell line candidates are identified more efficiently and are scaled up for further evaluation in a more directed, streamlined manner, so that the desired production cell lines are moved forward for downstream processing with greater accuracy and less upstream effort.

## Claims

1. A method for selecting a producer host cell stably expressing a target polypeptide at a high level, the method comprising:
(a) contacting at least one recombinant eukaryotic host cell containing a recombinant polynucleotide, wherein the recombinant polynucleotide comprises a promoter element; a polynucleotide encoding a cell surface marker polypeptide; and a polynucleotide encoding a target polypeptide, wherein the polynucleotide encoding the cell surface marker polypeptide includes an alternate start codon, wherein the cell surface marker polynucleotide and the target polynucleotide are transcribed on the same mRNA, and wherein the host cell is cultured under conditions such that the cell surface marker polypeptide is expressed on the surface of the host cell, with a detectable agent that recognizes and directly or indirectly binds the cell surface marker if present on the surface of the host cell, said contacting being under conditions that favour binding of the agent with the cell surface marker;
(b) selecting one or more host cells that are directly or indirectly bound to the agent;
(c) preparing one or more clonal populations of the host cells from step (b) and;
(d) analyzing one or more clonal populations from step (c) by detecting the level of the cell surface marker expression on said clonal populations and selecting one or more clonal populations with a high expression level of the cell surface marker, thereby selecting one or more clonal populations stably expressing the target polypeptide.

2. An assembly to select a recombinant eukaryotic host cell that stably and highly expresses a target polypeptide, the assembly comprising the following components:
(a) at least one recombinant eukaryotic host cell containing a recombinant polynucleotide, wherein the recombinant polynucleotide comprises a promoter element; a polynucleotide encoding a cell surface marker polypeptide; and a polynucleotide encoding a target polypeptide; wherein the polynucleotide encoding the cell surface marker polypeptide includes an alternate start codon; and wherein the polynucleotide encoding the cell surface marker polypeptide and the polynucleotide encoding the target polypeptide are transcribed on the same mRNA;
(b) an agent that directly or indirectly binds the cell surface marker polypeptide; and
(c) a means for detecting the agent when it is directly or indirectly bound to the cell surface marker polypeptide.

3. The method of Claim 1, wherein step (b) and/or step (d) is performed by florescence activated cell sorting.

4. The method of Claim 1, wherein step (d) is performed by florescence activated cell sorting and wherein the florescence activated cell sorting profile of a clonal population selected at step (d) indicates a narrow distribution around the mean, thereby indicating a clonal population stably expressing the target polypeptide.

5. The method of Claim 1, wherein step (d) is performed 7-28 days after step (c).

6. The method of Claim 1, wherein the high expression level is a level of expression of the cell surface marker that is higher than the expression level of at least 50, 70, 80, 90, or 95 or 99% of the cells analyzed in step (b) and/or step (d).

7. The method of Claim 1 or the assembly of Claim 2, wherein the promoter is a eukaryotic promoter.

8. The method of Claim 1 or the assembly of Claim 2, wherein the cell surface marker polynucleotide encodes a cell surface marker other than a CD4 polypeptide or wherein the cell surface marker polynucleotide encodes a cell surface marker selected from the group consisting of CD20, CD52 and CD59.

9. The method of Claim 1 or the assembly of Claim 2, wherein the target polypeptide comprises a therapeutic polypeptide or a therapeutic protein.

10. The method of Claim 1 or the assembly of Claim 2, wherein the promoter is located upstream to the polynucleotide encoding the cell surface marker polypeptide, or upstream to the polynucleotide encoding the target polypeptide.

11. The method of Claim 1 or the assembly of Claim 2, wherein the promoter is operatively linked to the polynucleotide encoding the cell surface marker polypeptide or operatively linked to the polynucleotide encoding the target polypeptide.

12. The method of Claim 1 or the assembly of Claim 2, wherein the recombinant polynucleotide further comprises a polynucleotide that increases or enhances transcription.

13. The method of Claim 1 or the assembly of Claim 2, wherein the eukaryotic host cell further comprises a second recombinant polynucleotide encoding a second target polypeptide under the control of a second promoter element.

14. The method of Claim 1 or the assembly of Claim 2, wherein the target polypeptide is selected from the group comprising an antibody, an antibody fragment, a hormone, an enzyme, a receptor protein, a lysosomal storage disorder protein, α-galactosidase A, and acid α-glucosidase.

15. The method of Claim 1 or the assembly of Claim 2, wherein the eukaryotic host cell is a yeast cell or a mammalian cell.

16. The assembly of Claim 2, further comprising:
(d) a means for separating at least one recombinant cell having the agent directly or indirectly bound to the cell surface marker.

17. The assembly of Claim 16, further comprising:
(e) a means to grow the at least one recombinant cell separated by the means of component (d).

18. The assembly of Claim 2, wherein the agent of component (b) is a detectably labelled antibody that directly or indirectly binds the cell surface marker polypeptide encoded by the polynucleotide.

19. The assembly of Claim 2, wherein the means of component (c) is a device for conducting flow cytometry.

## Patentansprüche

1. Verfahren zur Auswahl einer produzierenden Wirtszelle, welche stabil ein Targetpolypeptid auf einem hohen Level exprimiert, wobei das Verfahren umfasst:
(a) In-Kontakt-bringen bringen mindestens einer rekombinanten eukaryotischen Wirtszelle, welche ein rekombinantes Polynukleotid enthält, wobei das rekombinante Polynukleotid ein Promotorelement; ein Polynukleotid, welches ein Zelloberflächenmarkerpolypeptid kodiert; und ein Polynukleotid, welches ein Targetpolypeptid kodiert, wobei das Polynukleotid, welches das Zelloberflächenmarkerpolypeptid kodiert, ein alternierendes Startcodon beinhaltet, wobei das Zelloberflächenmarkerpolynukleotid und das Targetpolynukleotid auf die gleiche mRNA transkribiert werden, und wobei die Wirtszelle unter derartigen Bedingungen kultiviert wird, so dass das Zelloberflächenmarkerpolypeptid auf der Oberfläche der Wirtszelle, mit einem detektierbaren Agens exprimiert wird, das den Zelloberflächenmarker erkennt und direkt oder indirekt daran bindet, falls auf der Oberfläche der Wirtszelle vorhanden, wobei das In-Kontakt-bringen unter Bedingungen, welche die Bindung des Agens mit dem Zelloberflächenmarker begünstigen, stattfindet;
(b) Auswählen einer oder mehrerer Wirtszellen, welche direkt oder indirekt an das Agens gebunden sind;
(c) Vorbereiten einer oder mehrerer klonaler Populationen der Wirtszellen aus Schritt (b) und;
(d) Analysieren einer oder mehrerer klonaler Populationen aus Schritt (c) durch Detektieren des Grades der Zelloberflächenmarkerexprimierung auf den klonalen Populationen und Auswahl einer oder mehrerer klonaler Populationen mit einem hohen Exprimierungsgrad des Zelloberflächenmarkers, wodurch eine oder mehrere klonale Populationen ausgewählt werden, welche stabil das Targetpolypeptid exprimieren.

2. Anordnung zur Auswahl einer rekombinanten eukaryotischen Wirtszelle, welche stabil und in hohem Maße ein Targetpolypeptid exprimiert, wobei die Anordnung folgende Komponenten umfasst:
(a) mindestens eine rekombinante eukaryotische Wirtszelle, enthaltend ein rekombinantes Polynukleotid, wobei das rekombinante Polynukleotid ein Promotorelement; ein Polynukleotid, welches ein Zelloberflächenmarkerpolypeptid kodiert; und ein Polynukleotid, welches ein Targetpolypeptid kodiert, umfasst, wobei das Polynukleotid, welches das Zelloberflächenmarkerpolypeptid kodiert, ein alternierendes Startcodon beinhaltet; und wobei das Polynukleotid, welches das Zelloberflächenmarkerpolypeptid kodiert und das Polynukleotid, welches das Targetpolypeptid kodiert, auf die gleiche mRNA transkribiert werden;
(b) ein Agens, das direkt oder indirekt das Zelloberflächenmarkerpolypeptid bindet; und
(c) ein Mittel zum Detektieren des Agens, wenn es direkt oder indirekt an das Zelloberflächenmarkerpolypeptid gebunden ist.

3. Verfahren gemäß Anspruch 1, wobei Schritt (b) und/oder Schritt (d) durch fluoreszenzaktivierte Zellsortierung durchgeführt werden/wird.

4. Verfahren gemäß Anspruch 1, wobei Schritt (d) durch fluoreszenaktivierte Zellsortierung durchgeführt wird und wobei das fluoreszenzaktivierte Zellsortierungsprofil einer bei Schritt (d) ausgewählten klonalen Population, eine enge Verteilung um den Mittelwert anzeigt, wodurch eine klonale Population angezeigt wird, welche stabil das Targetpolypeptid exprimiert.

5. Verfahren gemäß Anspruch 1, wobei Schritt (d) 7 bis 28 Tage nach Schritt (c) durchgeführt wird.

6. Verfahren gemäß Anspruch 1, wobei der hohe Exprimierungsgrad ein Grad der Exprimierung des Zelloberflächenmarkers ist, welcher höher als der Exprimierungsgrad von mindestens 50, 70, 80, 90, oder 95 oder 99 % der in Schritt (b) und/oder Schritt (d) analysierten Zellen, ist.

7. Verfahren gemäß Anspruch 1 oder Anordnung gemäß Anspruch 2, wobei der Promotor ein eukaryotischer Promotor ist.

8. Verfahren gemäß Anspruch 1 oder Anordnung gemäß Anspruch 2, wobei das Zelloberflächenmarkerpolynukleotid einen anderen Zelloberflächenmarker als ein CD4-Polypeptid kodiert, oder wobei das Zelloberflächenmarkerpolynukleotid einen Zelloberflächenmarker kodiert, ausgewählt aus der Gruppe bestehend aus CD20, CD52 und CD59.

9. Verfahren gemäß Anspruch 1 oder Anordnung gemäß Anspruch 2, wobei das Targepolypeptid eine therapeutisches Polypeptid oder ein therapeutisches Protein umfasst.

10. Verfahren gemäß Anspruch 1 oder Anordnung gemäß Anspruch 2, wobei der Promotor oberhalb des Polynukleotids, welches das Zelloberflächenmarkerpolypeptid kodiert, oder oberhalb des Polynukleotids, welches das Targetpolypeptid kodiert, ist.

11. Verfahren gemäß Anspruch 1 oder Anordnung gemäß Anspruch 2, wobei der Promotor mit dem Polynukleotid, welches das Zelloberflächenmarkerpolypeptid kodiert oder mit dem Polynukleotid, welches das Targetpolypeptid kodiert, operabel (operatively) verbunden ist.

12. Verfahren gemäß Anspruch 1 oder Anordnung gemäß Anspruch 2, wobei das rekombinante Polynukleotid ferner ein Polynukleotid umfasst, das die Transkription erhöht oder verstärkt.

13. Verfahren gemäß Anspruch 1 oder Anordnung gemäß Anspruch 2, wobei die eukaryotische Wirtszelle ferner ein zweites rekombinantes Polynukleotid umfasst, welches ein zweites Targetpolypeptid unter der Kontrolle eines zweiten Promotors kodiert.

14. Verfahren gemäß Anspruch 1 oder Anordnung gemäß Anspruch 2, wobei das Targetpolypeptid ausgewählt ist aus der Gruppe umfassend, einen Antikörper, ein Antikörperfragment, ein Hormon, ein Enzym, ein Rezeptorprotein, ein lysosomales Speichererkrankungsprotein, α-Galactosidase A, und saure α-Galactosidase.

15. Verfahren gemäß Anspruch 1 oder Anordnung gemäß Anspruch 2, wobei die eukaryotische Wirtszelle eine Hefezelle oder eine Säugetierzelle ist.

16. Anordnung gemäß Anspruch 2, ferner umfassend:
(d) Mittel zur Trennung von mindestens einer rekombinanten Zelle, welche das Agens direkt oder indirekt an den Zelloberflächenmarker gebunden hat.

17. Anordnung gemäß Anspruch 16, ferner umfassend:
(e) Mittel zum Wachstum der, mindestens einer, rekombinanten Zelle, welche durch die Mittel der Komponente (d) abgetrennt wurde.

18. Anordnung gemäß Anspruch 2, wobei das Agens der Komponente (b) ein detektierbar markierter Antikörper ist, welcher direkt oder indirekt das Zelloberflächenmarkerpolypeptid bindet, welches durch das Polynukleotid kodiert wurde.

19. Anordnung gemäß Anspruch 2, wobei das Mittel der Komponente (c) eine Vorrichtung zur Durchführung von Flusszytometrie ist.

## Revendications

1. Procédé de sélection d'une cellule hôte productrice exprimant de manière stable un polypeptide cible à un taux élevé, le procédé comprenant :
(a) la mise en contact d'au moins une cellule hôte eucaryote recombinante contenant un polynucléotide recombinant, où le polynucléotide recombinant comprend un élément promoteur ; un polynucléotide codant pour un polypeptide marqueur de la surface cellulaire ; et un polynucléotide codant pour un polypeptide cible, où le polynucléotide codant pour le polypeptide marqueur de la surface cellulaire comprend un codon d'initiation alternatif, où le polynucléotide du marqueur de la surface cellulaire et le polynucléotide cible sont transcrits sur le même ARNm, et où la cellule hôte est cultivée dans des conditions telles que le polypeptide marqueur de la surface cellulaire est exprimé sur la surface de la cellule hôte, avec un agent détectable qui reconnaît et se lie directement ou indirectement au marqueur de la surface cellulaire s'il est présent sur la surface de la cellule hôte, ladite mise en contact étant réalisée dans des conditions qui favorisent la liaison de l'agent au marqueur de la surface cellulaire ;
(b) la sélection d'une ou plusieurs cellules hôtes qui sont liées directement ou indirectement à l'agent ;
(c) la préparation d'une ou plusieurs populations clonales des cellules hôtes issues de l'étape (b) et ;
(d) l'analyse d'une ou plusieurs populations clonales issues de l'étape (c) par la détection du taux d'expression du marqueur de la surface cellulaire sur lesdites populations clonales et la sélection d'une ou plusieurs populations clonales présentant un taux d'expression élevé du marqueur de la surface cellulaire, sélectionnant de cette manière une ou plusieurs populations clonales exprimant de manière stable le polypeptide cible.

2. Assemblage pour sélectionner une cellule hôte eucaryote recombinante qui exprime de manière stable et élevée un polypeptide cible, l'assemblage comprenant les composantes suivantes :
(a) au moins une cellule hôte eucaryote recombinante contenant un polynucléotide recombinant, où le polynucléotide recombinant comprend un élément promoteur ; un polynucléotide codant pour un polypeptide marqueur de la surface cellulaire ; et un polynucléotide codant pour un polypeptide cible, où le polynucléotide codant pour le polypeptide marqueur de la surface cellulaire comprend un codon d'initiation alternatif ; et où le polynucléotide codant pour le polypeptide marqueur de la surface cellulaire et le polynucléotide codant pour le polypeptide cible sont transcrits sur le même ARNm ;
(b) un agent qui se lie directement ou indirectement au polypeptide marqueur de la surface cellulaire ; et
(c) un moyen de détection de l'agent lorsqu'il est lié directement ou indirectement au polypeptide marqueur de la surface cellulaire.

3. Procédé selon la revendication 1, dans lequel l'étape (b) et/ou l'étape (d) est réalisée par tri cellulaire activé par fluorescence.

4. Procédé selon la revendication 1, dans lequel l'étape (d) est réalisée par tri cellulaire activé par fluorescence et où le profil du tri cellulaire activé par fluorescence d'une population clonale sélectionnée à l'étape (d) indique une distribution étroite autour de la moyenne, indiquant de cette manière une population clonale exprimant de manière stable le polypeptide cible.

5. Procédé selon la revendication 1, dans lequel l'étape (d) est réalisée 7 à 28 jours après l'étape (c).

6. Procédé selon la revendication 1, dans lequel le taux d'expression élevé est un taux d'expression du marqueur de la surface cellulaire qui est supérieur au taux d'expression d'au moins 50, 70, 80, 90 ou 95 ou 99 % des cellules analysées dans l'étape (b) et/ou l'étape (d).

7. Procédé selon la revendication 1 ou assemblage selon la revendication 2, dans lequel le promoteur est un promoteur eucaryote.

8. Procédé selon la revendication 1 ou assemblage selon la revendication 2, dans lequel le polynucléotide du marqueur de la surface cellulaire code pour un marqueur de la surface cellulaire autre qu'un polypeptide CD4 ou bien où le polynucléotide du marqueur de la surface cellulaire code pour un marqueur de la surface cellulaire choisi dans le groupe constitué par CD20, CD52 et CD59.

9. Procédé selon la revendication 1 ou assemblage selon la revendication 2, dans lequel le polypeptide cible comprend un polypeptide thérapeutique ou une protéine thérapeutique.

10. Procédé selon la revendication 1 ou assemblage selon la revendication 2, dans lequel le promoteur est localisé en amont du polynucléotide codant pour le polypeptide marqueur de la surface cellulaire, ou en amont du polynucléotide codant pour le polypeptide cible.

11. Procédé selon la revendication 1 ou assemblage selon la revendication 2, dans lequel le promoteur est lié de manière fonctionnelle au polynucléotide codant pour le polypeptide marqueur de la surface cellulaire ou lié de manière fonctionnelle au polynucléotide codant pour le polypeptide cible.

12. Procédé selon la revendication 1 ou assemblage selon la revendication 2, dans lequel le polynucléotide recombinant comprend en outre un polynucléotide qui augmente ou amplifie la transcription.

13. Procédé selon la revendication 1 ou assemblage selon la revendication 2, dans lequel la cellule hôte eucaryote comprend en outre un second polynucléotide recombinant codant pour un second polypeptide cible sous le contrôle d'un second élément promoteur.

14. Procédé selon la revendication 1 ou assemblage selon la revendication 2, dans lequel le polypeptide cible est choisi dans le groupe comprenant un anticorps, un fragment d'anticorps, une hormone, une enzyme, une protéine récepteur, une protéine de trouble de stockage lysosomial, l'α-galactosidase A et l'α-glucosidase acide.

15. Procédé selon la revendication 1 ou assemblage selon la revendication 2, dans lequel la cellule hôte eucaryote est une cellule de levure ou une cellule mammalienne.

16. Assemblage selon la revendication 2, comprenant en outre :
(d) un moyen de séparation d'au moins une cellule recombinante ayant l'agent lié directement ou indirectement au marqueur de la surface cellulaire.

17. Assemblage selon la revendication 16, comprenant en outre :
(e) un moyen de culture de la au moins une cellule recombinante séparée par le moyen de la composante (d).

18. Assemblage selon la revendication 2, dans lequel l'agent de la composante (b) est un anticorps marqué de manière détectable qui se lie directement ou indirectement au polypeptide marqueur de la surface cellulaire codé par le polynucléotide.

19. Assemblage selon la revendication 2, dans lequel le moyen de la composante (c) est un dispositif de conduction d'une cytométrie en flux.
